# EUROPÄISCHE PATENTANMELDUNG

(11) **EP 3 203 255 A1**
(43) Veröffentlichungstag der Anmeldung: **09.08.2017**
(21) Anmeldenummer: 16153815.2
(22) Anmeldetag: 02.02.2016
(51) Int. Cl.: G01R 33/56, G01R 33/563, A61B 5/0275, A61M 5/00

(54) **BESTIMMUNG DER MINDESTDAUER EINES KONTRASTMITTELBOLUS UND LIMITIERUNG VON INJEKTIONSRATEN**

(71) Anmelder: Bayer Pharma Aktiengesellschaft, 13353 Berlin (DE)
(72) Erfinder: Rohrer, Martin, 12203 Berlin (DE)
(74) Vertreter: BIP Patents

(57) **Zusammenfassung**

Die Erfindung betrifft ein Verfahren zur Vorbestimmung einer maximal möglichen Bolusapplikationsdauer (T_{Bmax}[T_{Amin}]) zur Erreichung einer minimal möglichen Bolusantwortdauer (T_{Amin}) einer Kontrastmittelbolusantwort (K(t)) an einer zweiten Gefäßposition (P2) in einer Untersuchungsumgebung (ROI) eines Patienten (P) an einer ersten Gefäßposition (P1) für ein kontrastmittelgestütztes bildgebendes Aufnahmeverfahren, wobei eine Korrelation zwischen der maximal möglichen Bolusapplikationsdauer (T_{Bmax}[T_{Amin}]) für eine Patientengruppe mit bekannten Patientenparametern (P_{P}) und mindestens einem Blutflussparameter (P_{B}) ermittelt wird.

Die Erfindung betrifft auch ein Verfahren zur Bestimmung einer optimalen Flussrate (F) für eine kürzest mögliche Bolusapplikation , wobei eine maximalmögliche Bolusapplikationsdauer (T_{Bmax}[Tₘᵢₙ]) zur Erreichung einer minimal möglichen Bolusantwortdauer (T_{Amin}) ermittelt, und hieraus die notwendige Flussrate (F) zur Applikation einer vorgegebenen zu applizierenden Kontrastmittelmenge (M_{KM}) bestimmt wird.

Außerdem betrifft die Erfindung ein Magnetresonanzsystem (1) zur Durchführung der o.g. Verfahren.

## Beschreibung

Verfahren zur Bestimmung einer Mindestdauer eines Kontrastmittelbolus und Limitierung von Injektionsraten, Verfahren zur Bestimmung der optimalen Flussrate bei einer Bolusapplikation und ein Magnetresonanzsystem

Die Erfindung betrifft ein Verfahren zur Vorbestimmung einer maximalen Bolusapplikationsdauer zur Erreichung einer minimalen Bolusantwortdauer einer Kontrastmittelbolusantwort an einer zweiten Gefäßposition in einer Untersuchungsumgebung eines Patienten nach einer automatisierten Bolusapplikation mit einem Kontrastmittelapplikator an einer ersten Gefäßposition für ein kontrastmittelgestütztes bildgebendes Aufnahmeverfahren.

Weiterhin betrifft die Erfindung ein Verfahren zur Bestimmung einer optimalen Flussrate für eine kürzest mögliche automatisierte Bolusapplikation eines Kontrastmittels mit einem Kontrastmittelapplikator für eine kontrastmittelgestützte Aufnahme eines Patienten auf der Basis einer zuvor ermittelten maximalen Bolusapplikationsdauer zur Erreichung einer minimalen Bolusantwortdauer.

Außerdem betrifft die Erfindung ein Verfahren zur Vermeidung einer fehlerhaften Einstellung der Flussrate und/oder Bolusapplikationsdauer eines Kontrastmittelapplikators, der einen Prozessor und einen Speicher aufweist oder damit verbunden ist, um mindestens ein im Speicher vorliegendes Computerprogramm im Betrieb auszuführen.

Neben den oben genannten Verfahren betrifft die Erfindung auch ein Magnetresonanzsystem zur Erzeugung einer kontrastmittelunterstützten MR-Aufnahme mit einem, von einem prozessorgesteuerten Kontrastmittelinjektor, wobei Computer und ein Speicher für Computerprogramme zur Durchführung dieser Verfahren vorliegen.

In der diagnostischen Bildgebung sind Verfahren der Magnetresonanztomographie (MRT) allgemein bekannt. Insbesondere zur Darstellung von Blutgefäßen in der Magnetresonanz-Angiographie (MRA) wird zur Hervorhebung der arteriellen Gefäße gegenüber dem restlichen Gewebe ein Kontrastmittel eingesetzt. Solche Verfahren werden als kontrastmittelverstärkte MR-Angiographie (CE-MRA) bezeichnet. Werden Darstellungen von peripher gelegenen Blutgefäßen, wie beispielsweise Beinarterien, aufgenommen, spricht man von kontrastmittelverstärkter peripherer MR-Angiographie (CE-pMRA).

Die CE-MRA stellt im Vergleich zu anderen, nicht durch Kontrastmittel verstärkten Bildgebungsmethoden, in vielen klinischen Fällen die bevorzugte Methode einer strahlungsfreien, nicht-invasiven Gefäßdiagnostik dar. Sie wurde vor ca. 20 Jahren erstmals durchgeführt, seitdem kontinuierlich technisch verbessert und stellt heute in den meisten Ländern ein fortgeschrittenes diagnostisches Standardverfahren dar.

Die klinischen Fragestellungen erfordern in den meisten Fällen eine gegenüber dem umliegenden Gewebe möglichst kontrastreiche Visualisierung des arteriellen Gefäßsystems, wobei die gleichzeitige Visualisierung des venösen Gefäßsystems in der Regel als störende Überlagerung gesehen wird. Bei solchen kontrastmittelgestützten MR-Untersuchungen, bei denen nur die erste Anflutungsphase des Kontrastmittels zur Bilddarstellung genutzt wird, also sogenannten "First Pass"-MR-Untersuchungen, ist es in vielen Fällen wesentlich, dass die Kontrastmittelanflutung, also die Bolusantwortdauer auf einen applizierten kurzen Kontrastmittelbolus auch möglichst kurz bleibt, um venöse Überlagerungen so gering wie möglich zu halten. Eine möglichst kurze Bolusantwortdauer ist insbesondere auch bei anderen, nicht-angiographischen Anwendungen, wie MRT-Perfusionsuntersuchungen beispielsweise im intrakraniellen Bereich von Vorteil.

Trotz erheblichen technischen Fortschritts während der letzten Jahre, bleibt bis heute die optimale zeitliche Synchronisation des Kontrastmittelbolus in der Zielregion (bolus timing) und die optimale Nutzung des injizierten Kontrastmittelbolus eine Herausforderung für das Personal, da große Erfahrung und grundlegendes physikalisches Verständnis des komplizierten bildgebenden MRT-Verfahrens gefordert sind.

Insbesondere der Wunsch zu einer kürzeren Dauer einer Kontrastmittelbolusantwort (Bolusantwortdauer) führt teilweise zu Problemen bei der Programmierung eines Kontrastmittelapplikators, da sich herausgestellt hat, dass bei kurzen Boluszeiten keineswegs noch eine unmittelbare Proportionalität zwischen der Bolusapplikationsdauer an einer ersten Gefäßposition und der Bolusantwortdauer an einer zweiten Gefäßposition vorliegt. Es hat sich hierbei gezeigt, dass es zu unnötig kurzen Injektionszeiten und damit zu unnötig hohen Injektionsraten kommen kann, die eine vermeidbare Belastung des Patienten, zum Beispiel durch zu groß gewählte Injektionskanülen, und eine Gefährdung an der i.V. - Injektionsstelle durch zu hohe Drücke darstellen können.

Neben dem Bedürfnis, den zeitlichen Verlauf der Kontrastmittelkonzentration nach der Gabe eines Bolus möglichst genau vorhersagen zu können, ist es daher auch wesentlich, den Zusammenhang zwischen der Bolusapplikationsdauer und der Bolusantwortdauer so zu verstehen, dass eine möglichst kurze Bolusantwortdauer erreicht werden kann, ohne den Kontrastmittelzugang bei der Applikation mehr als notwendig zu gefährden.

Es ist daher Aufgabe der Erfindung, ein Verfahren zur Ermöglichung der Vorbestimmung einer maximal erreichbaren Bolusapplikationsdauer bei gleichzeitig minimaler Bolusantwortdauer und davon ausgehend ein Verfahren zur Bestimmung einer optimalen Flussrate für eine kürzest mögliche Bolusapplikation eines Kontrastmittels, ein Verfahren zur Vermeidung einer fehlerhaften Einstellung der Flussrate und/oder Bolusapplikationsdauer eines Kontrastmittelapplikators und ein Magnetresonanzsystem hierfür zu finden.

Diese Aufgabe wird durch die Merkmale der unabhängigen Patentansprüche gelöst. Vorteilhafte Weiterbildungen der Erfindung sind Gegenstand untergeordneter Ansprüche.

Die Erfinder haben erkannt, dass es bei einer Kontrastmittelbolusapplikation an einer ersten Gefäßposition, meist im Bereich einer Armvene, zur Erzeugung einer möglichst kurzen Bolusantwortdauer, als einer möglichst kurzen Erhöhung der Kontrastmittelkonzentration im Blutfluss, an einer anderen, meist arteriellen, Gefäßposition im Kreislaufsystem eines Patienten, nicht zielführend ist, die Bolusapplikationsdauer immer weiter zu reduzieren, da sich die erreichbare minimale Bolusantwortdauer nicht beliebig reduzieren lässt. Vielmehr nähert sich die Bolusantwortdauer mit kürzer werdendem Bolus einem Grenzwert einer minimalen Bolusapplikationsdauer, der - bei sonst konstanten Bedingungen - nicht zu unterschreiten ist. Entsprechend ist auch ein Bolusprofil, bei dem in immer kürzerer Zeit mit immer höher werdendem Druck die gleiche Menge Kontrastmittel injiziert wird, kontraproduktiv, da durch die dann momentan hohe Injektionsmenge Läsionen an der Vene entstehen können. Auch kann es sein, dass durch zu hohe Flussraten und insbesondere Flussgeschwindigkeiten des injizierten Kontrastmittels Strömungseffekte ausgelöst werden, die eher zu einer ungewollten Verbreiterung der Bolusantwortdauer führen als zu einer Verkürzung der Bolusantwortdauer.

Zusätzlich haben die Erfinder auch erkannt, dass die minimal erreichbare Bolusantwortdauer bei einer hierfür maximalen Bolusdauer - genauer Bolusapplikationsdauer - mit mindestens einem Blutflussparameter korreliert. Als Blutflussparameter kann dabei eine Blutflusseigenschaft oder eine Funktion aus einer oder mehreren Blutflusseigenschaften verwendet werden, wobei die Blutflusseigenschaften gegebenenfalls ohne die Anwesenheit von Kontrastmittel im Blutkreislauf, zum Beispiel durch eine Phasenkontrast-Magnetresonanz-Messung bestimmt werden kann. Der einfachste Fall eines solchen Blutflussparameters ist dabei die unmittelbare Verwendung einer mittleren Blutflussgeschwindigkeit an einer vorgegebenen relevanten Gefäßposition als Blutflusseigenschaft.

Aufgrund dieser Erkenntnis der Korrelation zwischen kontrastmittelfrei bestimmbarem Blutflussparameter und der minimal erreichbaren Bolusantwortdauer bei hierfür maximaler Bolusapplikationsdauer besteht nun die Möglichkeit, einen optimalen Kontrastmittelbolus zu applizieren, bei dem sich die minimal erreichbare Bolusantwortzeit einstellt, wobei gleichzeitig auch kontraproduktive überhöhte Flussgeschwindigkeiten und Flussraten vermieden werden können. Da dabei keine vorhergehende kontrastmittelunterstützte Messung notwendig ist, befindet sich zum Zeitpunkt der ersten Anflutung bei der Messung kein weiteres Kontrastmittel im Blutkreislauf, so dass ein optimales Kontrast-zu-Rausch-Verhalten bei der Bilderzeugung und damit eine optimale Bildqualität erreicht wird.

Gemäß einem weiteren Aspekt der Erfindung kann diese Kenntnis auch dazu verwendet werden, eine Fehleinstellung an einem Kontrastmittelinjektor zu vermeiden, beziehungsweise die optimale Einstellung von Flussrate und Bolusapplikationsdauer zu finden.

Demgemäß schlagen die Erfinder ein Verfahren zur Vorbestimmung einer maximalen Bolusapplikationsdauer (=T_{Bmax}[T_{Amin}]) zur Erreichung einer minimalen Bolusantwortdauer (=T_{Amin}) einer Kontrastmittelbolusantwort an einer zweiten Gefäßposition in einer Untersuchungsumgebung (=ROI) eines Patienten nach einer automatisierten Bolusapplikation mit einem Kontrastmittelapplikator an einer ersten Gefäßposition für ein kontrastmittelgestütztes bildgebendes Aufnahmeverfahren vor, wobei die folgenden Verfahrensschritte ausgeführt werden:
- Ermittlung einer Korrelation zwischen der maximalen Bolusapplikationsdauer für eine Patientengruppe mit mindestens einem bekannten Patientenparameter einerseits und mindestens einem Blutflussparameter andererseits, wobei der Blutflussparameter von mindestens einer Blutflusseigenschaft an einer dritten Gefäßposition abhängig ist,
- Ausgabe einer Tabelle (=LUT) oder Funktion der Korrelation und/oder Speicherung einer Tabelle oder Funktion der Korrelation in einem elektronischen Speicher zur weiteren Verwendung durch einen Computer.

Durch die Zurverfügungstellung einer solchen Korrelations-Tabelle oder Korrelations-Funktion, in der als Variable einerseits die verschiedenen Patientenparameter berücksichtigt sind, wodurch jeweils miteinander vergleichbare Patienten und Patientengruppen betrachtet werden, und andererseits der Blutflussparameter, aus denen sich dann die zugehörige maximale Bolusapplikationsdauer des jeweiligen Patienten zur Erreichung einer minimalen Bolusantwortdauer ablesen oder berechnen lässt, wird dem Fachpersonal ein Mittel zur Verfügung gestellt, mit dem unnötige, zeitraubende und für die Patienten belastende Versuche zur Erreichung einer minimalen Bolusantwortzeit in einem Untersuchungsbereich vermieden werden.

Vorzugsweise kann als zweite Gefäßposition und/oder Untersuchungsumgebung ein Bereich gewählt werden, der sich strömungsbezogen hinter der Herz-LungenPassage, zum Beispiel im Bereich der Karotiden und des Kopfes, befindet. In den meisten Fällen wird als erste Gefäßposition eine Position in der Armvene gewählt werden.

Da die mindestens eine Blutflusseigenschaft auch unter Abwesenheit von Kontrastmittel, vorzugsweise durch eine Phasenkontrast-Magnetresonanz-Messung, ermittelt werden kann, entsteht durch diese Messungen auch keine Gabe von Kontrastmittel in den Blutkreislauf, so dass bei nachfolgenden Messungen bei einer ersten Anflutung auch kein "Hintergrundrauschen" durch Kontrastmittelreste im Blutkreislauf aus vorherigen Messungen zu befürchten ist und damit bei der eigentlichen Messung ein verbessertes Kontrast-zu-Rausch-Verhältnis erreicht werden kann.

Bezüglich der zu verwendenden Patientenparameter zur Vergleichbarmachung des getesteten Patientenklientels und der daraus gewonnen Ergebnisse mit einem aktuell zu untersuchenden Patienten wird vorgeschlagen, dass mindestens ein Patientenparameter, vorzugsweise mehrere Patientenparameter, der nachfolgenden Liste verwendet wird/werden:
- Geschlecht,
- Gewicht,
- Größe,
- Alter,
- Herzfrequenz,
- BMI,
- Typisierung der Statur,
- Abstand zwischen vorgegebenen Gefäßpositionen.

Als vorteilhaft zu untersuchende Blutflusseigenschaften wird vorgeschlagen, dass als mindestens eine Blutflusseigenschaft mindestens eine der folgenden Eigenschaften verwendet wird:
- maximale, minimale oder mittlere Blutflussgeschwindigkeit an mindestens einer vorgegebenen Position im Gefäßquerschnitt an der Gefäßposition;
- maximale, minimale oder mittlere Blutflussgeschwindigkeit an mindestens einer vorgegebenen Position im Gefäßquerschnitt an der Gefäßposition zu einer vorgegebenen Herz- oder Pulsphase;
- maximale, minimale oder mittlere Blutflussgeschwindigkeit an mindestens einer vorgegebenen Position im Gefäßquerschnitt an der Gefäßposition zu einer vorgegebenen Herz- oder Pulsphase über einen vorgegebenen Messzeitraum;
- maximales, minimales oder mittleres Blutflussvolumen an mindestens einer vorgegebenen Position im Gefäßquerschnitt an der Gefäßposition;
- maximale, minimale oder mittlere Blutflussvolumen an mindestens einer vorgegebenen Position im Gefäßquerschnitt an der Gefäßposition zu einer vorgegebenen Herz- oder Pulsphase;
- maximale, minimale oder mittlere Blutflussvolumen an mindestens einer vorgegebenen Position im Gefäßquerschnitt an der Gefäßposition zu einer vorgegebenen Herz- oder Pulsphase über einen vorgegebenen Messzeitraum;
- eine geometrische Eigenschaft eines Geschwindigkeitsprofils über den Gefäßquerschnitt an der Gefäßposition;
- Nettovorwärtsvolumen über einen vorgegebenen Zeitraum oder pro Herzschlag.

In einer besonders einfachen Ausführungsform des Verfahrens kann als Blutflussparameter die Blutflusseigenschaft selbst verwendet werden.

Weiterhin kann als Blutflussparameter eine absolute oder prozentuale Differenz zwischen der Blutflusseigenschaft an der dritten Gefäßposition zur gleichen Blutflusseigenschaft an einer vierten Gefäßposition verwendet werden.

Als günstige erste Gefäßposition sollte diese mindestens eine der nachfolgenden Ortsangaben oder Bedingungen erfüllen:
- die erste Gefäßposition liegt in einem venösen Gefäß,
- die erste Gefäßposition liegt in einer Armvene,
- die erste Gefäßposition liegt am Handrücken,
- die erste Gefäßposition liegt an einem zentralvenösen Katheter,
- die erste Gefäßposition liegt zwischen Handrücken und V. axillari,
- die erste Gefäßposition liegt zwischen Fuß und V. saphena magna
- die erste Gefäßposition liegt in einem zentralvenösen Gefäß.

Weiterhin wird vorgeschlagen, dass die zweite Gefäßposition mindestens eine der nachfolgenden Ortsangaben oder Bedingungen erfüllt:
- die zweite Gefäßposition liegt in einem arteriellen Gefäß,
- die zweite Gefäßposition liegt in einer Beinarterie,
- die zweite Gefäßposition liegt flussabwärts zur dritten Gefäßposition die zweite Gefäßposition liegt flussabwärts der Bifurcation,
- die zweite Gefäßposition liegt in einer peripheren Arterie, vorzugsweise im Kniebereich,
- die zweite Gefäßposition liegt im Armbereich,
- die zweite Gefäßposition liegt im Halsbereich,
- die zweite Gefäßposition liegt im Kopfbereich.

Die dritte Gefäßposition sollte mindestens eine der nachfolgenden Ortsangaben oder Bedingungen erfüllen:
- die dritte Gefäßposition ist die zweite Gefäßposition die dritte Gefäßposition liegt in der Aorta thorakalis,
- die dritte Gefäßposition liegt in der Aorta abdominalis,
- die dritte Gefäßposition liegt in der Aorta ascendens,
- die dritte Gefäßposition liegt zwischen Aorta thorakalis und die dritte Gefäßposition liegt vor der Bifurcation,
- die dritte Gefäßposition liegt zwischen Bifurcation und der zweiten Gefäßposition.

Schließlich wird für die vierte Gefäßposition mindestens eine der nachfolgenden Ortsangaben oder Bedingungen erfüllt:
- die vierte Gefäßposition liegt flussaufwärts der dritten Gefäßposition,
- die vierte Gefäßposition liegt flussabwärts zwischen der dritten Gefäßposition und der zweiten Gefäßposition,
- die vierte Gefäßposition liegt flussabwärts der zweiten Gefäßposition.

Wie oben dargestellt besteht also die Möglichkeit, vorzugsweise kontrastmittelfrei und ohne Belastung des Patienten mit ionisierender Strahlung, die maximale Bolusapplikationsdauer zur Erreichung einer minimal möglichen Bolusantwortdauer zu bestimmen. Auf der Basis einer solchen Kenntnis kann dann eine optimale Flussrate und Dauer einer automatisierten Bolusapplikation bestimmt werden, welche einerseits die Flussrate so niedrig als möglich hält und andererseits jedoch eine kürzest mögliche Bolusantwortdauer im Untersuchungsbereich erzeugt.

Demgemäß wird ein Verfahren zur Bestimmung einer optimalen Flussrate für eine kürzest mögliche automatisierte Bolusapplikation eines Kontrastmittels mit einem Kontrastmittelapplikator für eine kontrastmittelgestützte Aufnahme eines Patienten vorgeschlagen, welches zumindest die folgenden Verfahrensschritte aufweist:
- Ermittlung einer maximalen Bolusapplikationsdauer zur Erreichung einer minimalen Bolusantwortdauer einer Kontrastmittelbolusantwort an einer zweiten Gefäßposition in einer Untersuchungsumgebung eines Patienten nach einer automatisierten Bolusapplikation mit einem Kontrastmittelapplikator an einer ersten Gefäßposition,
- Berechnung einer an einem Kontrastmittelinjektor einzustellenden Flussrate für einen Kontrastmittelbolus bei der vorbestimmten maximalen Bolusapplikationsdauer zur Applikation einer vorgegebenen zu applizierenden Kontrastmittelmenge,
- Speicherung der berechneten Flussrate und der Bolusapplikationsdauer in einem Speicher des Kontrastmittelinjektors zur Verwendung für eine auszuführende Kontrastmittelbolusinjektion.

Hierbei können zur Ermittlung der Bolusapplikationsdauer mit T_{B=} T_{Bmax}[T_{Amin}] zumindest die folgenden Verfahrensschritte ausgeführt werden:
- Durchführung einer kontrastmittelfreien Messung mindestens einer Blutflusseigenschaft am aktuell zu untersuchenden Patienten,
- Bestimmung mindestens eines Blutflussparameters auf der Basis der gemessenen mindestens einen Blutflusseigenschaft,
- Ermittlung der maximalen Bolusapplikationsdauer zur Erreichung einer minimalen Bolusantwortdauer unter Anwendung einer vorbestimmten Korrelation zwischen dem mindestens einen Blutflussparameter und der maximalen Bolusapplikationsdauer unter Berücksichtigung mindestens eines Patientenparameters.

Vorzugsweise kann für die Ermittlung der Korrelation zwischen dem mindestens einen Blutflussparameter und der maximalen Bolusapplikationsdauer zur Erreichung einer minimalen Bolusantwortdauer das oben geschilderte Verfahren genutzt werden, wobei es selbstverständlich vorzuziehen ist, dass bei den Messungen zur Bestimmung der Korrelation und bei den späteren Messungen am aktuell zu untersuchenden Patienten die gleichen Blutflusseigenschaften an den gleichen Gefäßpositionen und damit die gleichen Blutflussparameter bestimmt werden, wobei das Referenzpatientenklientel mit seinen Patientenparameter möglichst weitgehend mit dem aktuell zu untersuchenden Patienten übereinstimmen sollte.

Das erfindungsgemäße Verfahren wird insbesondere dafür vorgeschlagen, damit eine Applikation des Kontrastmittels mit einem Kontrastmittelapplikator unter Anwendung der ermittelten Flussrate über die vorgegebene Bolusapplikationsdauer mit T_{B=} T_{Bmax}[T_{Amin}] erfolgt und zumindest eine Abbildung zumindest der Untersuchungsumgebung des Patienten durch ein kontrastmittelgestütztes bildgebendes Verfahren erzeugt wird. Vorzugsweise sollte die Erzeugung der mindestens einen Abbildung durch eine Magnetresonanz-Untersuchung oder mit einem Magnetresonanztomographie-System erfolgen.

Besonders vorteilhaft ist das beschriebene Verfahren für eine Untersuchung, bei der eine erste Anflutung von Kontrastmittel im Untersuchungsbereich abgebildet wird. Entsprechend wird vorgeschlagen, dass die kontrastmittelgestützte Erzeugung einer Aufnahme des Untersuchungsgebietes vorzugsweise im Kopf-/Gehirnbereich, nur während der ersten Anflutung des Kontrastmittels im Untersuchungsgebiet erfolgt.

Auf der Basis der Kenntnis der maximalen Bolusapplikationsdauer T_{Bmax}[T_{Amin}] wird außerdem auch ein Verfahren zur Vermeidung einer fehlerhaften Einstellung der Flussrate und/oder Bolusapplikationsdauer eines Kontrastmittelapplikators vorgeschlagen, wobei dieser einen Prozessor und einen Speicher aufweist oder damit verbunden ist, um mindestens ein im Speicher vorliegendes Computerprogramm im Betrieb auszuführen, und das mindestens eine Computerprogramm eine Ablaufroutine aufweist, durch welche sichergestellt wird, dass eine eingegebene Bolusapplikationsdauer gleich oder größer als eine zuvor ermittelte maximale Bolusapplikationsdauer zur Erreichung einer minimalen Bolusantwortdauer einer Kontrastmittelbolusantwort an einer zweiten Gefäßposition in einer Untersuchungsumgebung eines Patienten nach einer automatisierten Bolusapplikation mit einem Kontrastmittelapplikator an einer ersten Gefäßposition ist.

Vorzugsweise sollte auch bei diesem Verfahren die maximale Bolusapplikationsdauer zur Erreichung einer minimalen Bolusantwortdauer mit den gleichen Blutflussparametern und Patientenparametern am aktuellen Patienten an den gleichen Gefäßpositionen ermittelt werden, die auch zur Ermittlung der Korrelation verwendet werden, wobei bevorzugt die oben bereits vorgeschlagenen Positionen verwendet werden können.

Weiterhin kann auch hierbei die maximale Bolusapplikationsdauer zur Erreichung einer minimalen Bolusantwortdauer des aktuell untersuchten Patienten mit Hilfe einer kontrastmittelfreien Phasenkontrast-Magnetresonanz-Bestimmung mindestens einer Blutflusseigenschaft ermittelt werden, wobei vorzugsweise das oben beschriebene Verfahren ausgeführt wird.

Neben dem erfindungsgemäßen Verfahren schlagen die Erfinder auch ein bildgebendes medizinisches Untersuchungssystem, vorzugsweise ein Magnetresonanzsystem zur Erzeugung einer kontrastmittelunterstützten MR-Aufnahme vor, wobei dieses mit einem prozessorgesteuerten Kontrastmittelinjektor ausgestattet ist und wobei Computer und ein Speicher für Computerprogramme vorliegen, und Computerprogramme gespeichert sind, welche im Betrieb das erfindungsgemäße Verfahren ausführen.

Nachfolgend wird die Erfindung anhand der Figuren näher beschrieben, wobei nur die zum Verständnis der Erfindung notwendigen Merkmale dargestellt sind. Es zeigen im Einzelnen:
- FIG 1:: Darstellung eines erfindungsgemäßen MRT-Systems;
- FIG 2:: Darstellung eines Kreislaufs eines Patienten;
- FIG 3:: Darstellung eines Verlaufes einer Bolusapplikation an einer ersten Gefäßposition und des damit korrespondierenden Verlaufes einer Bolusantwort an einer zweiten Gefäßposition;
- FIG 4:: Darstellung von drei Bolusapplikationen unterschiedlicher Bolusapplikationsdauer an der gleichen ersten Gefäßposition und jeweils daraus sich ergebenden Bolusantwortverläufen an der gleichen zweiten Gefäßposition;
- FIG 5:: Darstellung einer Beziehung zwischen dem Bolus an einer ersten Gefäßposition mit der Bolusapplikationsdauer T_{B} und der Bolusantwortdauer T_{A} einer darauf folgenden Bolusantwort an einer zweiten Gefäßposition eines Patienten mit daraus resultierender minimalen Bolusantwortdauer T_{Amin} und hierfür möglichen maximalen Bolusdauer T_{Bmax}[T_{Amin}];
- FIG 6:: Darstellung der Korrelation zwischen einem Blutflussparameter P_{B} und einer minimalen Bolusantwortdauer T_{Amin} mit der hierfür möglichen maximalen Bolusdauer T_{Bmax}[T_{Amin}].

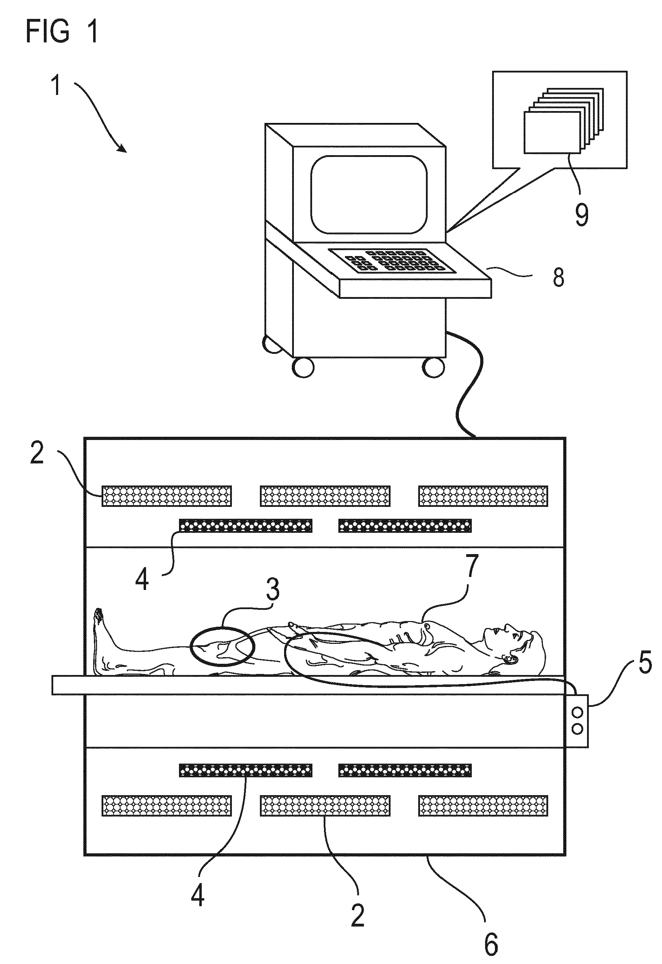

In der **Figur 1** ist schematisch ein Magnetresonanztomographie-System (MRT-System) 1 dargestellt. Bei diesem MRT-System 1 befinden sich in einem Gehäuse 6 Magnetspulen 2 zur Erzeugung eines starken magnetischen Hauptfeldes, wodurch sich die Wasserstoffkerne im Körper des Patienten 7 entsprechend ihrem Spin parallel oder anti-parallel zu den Magnetfeldlinien ausrichten. Durch Anregung der Atomkerne mit einem elektromagnetischen Wechselfeld in der Resonanzfrequenz der Atomkerne werden diese zur Schwingung veranlasst. Nach dem Ausschalten der Anregungsfrequenz kehren die Atomkerne wieder in ihre Lage zurück und geben ihre Schwingungsenergie in Form von elektromagnetischer Schwingungsenergie ab, die mit Hilfe von Empfangsspulen 3, welche möglichst in der Nähe einer zu betrachtenden ROI am Patienten 7 angeordnet werden, gemessen wird. Durch zusätzliche Magnetspulen 4 wird ein schwaches Magnetfeld mit einem definierten Feldgradienten erzeugt, wodurch die von den Kernen abgegebenen Signale Ortsinformationen erhalten, durch die die Position des abgegebenen Signals definierbar ist. Die Steuerung dieses Systems 1 und die Auswertung der Mess-Signale erfolgt durch die Steuer- und Recheneinheit 8, welche in ihrem Speicher Programme 9 aufweist, die im Betrieb neben der Steuerung und Bildberechnung auch das erfindungsgemäße Verfahren ausführen können.

Zur besseren Darstellung von Blutgefäßen ist es zum Teil notwendig, den Blutkreislauf des Patienten kurzfristig mit Kontrastmittel anzureichern, wozu meist ein Kontrastmittelinjektor 5 verwendet wird, der elektronisch gesteuert - entweder durch die Recheneinheit 8 oder durch einen separaten Prozessor - den Volumenfluss eines zur Messung zu applizierenden Kontrastmittels (=Bolus) erzeugt, wobei der Fluss (Volumen pro Zeit) beziehungsweise deren zeitlicher Verlauf und die Flussdauer T_{B} entsprechend vorbestimmt wird.

Mit Hilfe eines solchen MRT-Systems ist es auch unter Verwendung mehrerer Empfangsantennen möglich ohne die Applikation von Kontrastmittel Blutflusseigenschaften, wie beispielsweise Flussgeschwindigkeiten, Geschwindigkeitsprofile oder Volumenflüsse, zu erhalten. Diesbezüglich wird lediglich beispielhaft auf die Druckschrift DE 102013204994 A1 verwiesen.

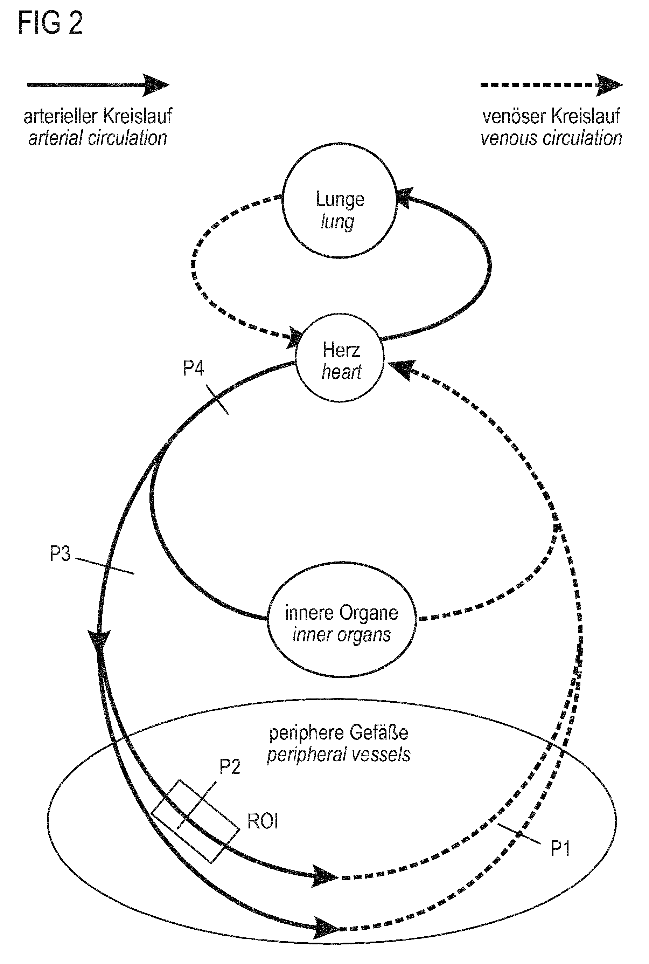

Zum besseren Verständnis der Erfindung wird in der **Figur 2** ein sehr schematisiert dargestellter Blutkreislauf eines Patienten gezeigt. Dieser geschlossene Kreislauf teilt sich in einen venösen Kreislauf (gestrichelte Linien) und einen arteriellen Kreislauf (durchgezogene Linien) auf und wird im Wesentlichen durch die Pumptätigkeit des Herzens betrieben. Im Lungenkreislauf ist das arterielle Blut - im Gegensatz zum sonstigen Blutkreislauf - sauerstoffarm und das venöse sauerstoffreich. Entsprechend einem solchen natürlichen Verlauf und den relativ einfach erzeugbaren und nutzbaren Zugängen im venösen Bereich, beispielsweise an einer Arm- oder Handvene, werden üblicherweise Kontrastmittel durch solche Zugänge, meist unter Zuhilfenahme von automatisch gesteuerten Kontrastmittelinjektoren appliziert. Entsprechend passiert ein dort gesetzter Bolus zunächst die nicht näher dargestellten rechten Herzkammern, wird über die Lungenpassage zu den ebenfalls nicht dargestellten linken Kammern des Herzens geleitet und gelangt von dort aus zu den Bereichen des Körpers, die mit einer MR-Aufnahmen abgebildet werden sollen und die bezüglich der Erfindung von Interesse sind, insbesondere auch in die peripheren Gefäßbereiche.

In der schematischen Darstellung der Figur 2 sind die wesentlichen Gefäßpositionen P1 bis P4 und der zumindest abzubildende Untersuchungsbereich ROI an beispielhaften und typischen Positionen markiert. Wie bereits erwähnt findet die Applikation in den meisten Fällen im Bereich des venösen Kreislaufes, entsprechend der eingetragenen Gefäßposition P1 an einem peripheren Gefäß, zum Beispiel einer Arm- oder Handvene, statt.

Das Gebiet von Interesse ROI, das bildgebend untersucht werden soll, kann - wie hier eingezeichnet - beispielsweise im Gebiet einer Beinarterie oder auch im Kopfbereich liegen, wo sich dann auch die Gefäßposition P2 befindet. Die weiteren Messpositionen, an denen dann Blutflusseigenschaften bestimmt werden, liegen in der Regel im arteriellen Gefäßsystem zwischen der linken Vorkammer des Herzens und der ROI. Es wird allerdings darauf hingewiesen, dass auch eine Positionierung der Messpunkte an Gefäßpositionen möglich ist, welche in Flussrichtung gesehen hinter der ROI oder P2 angeordnet sind.

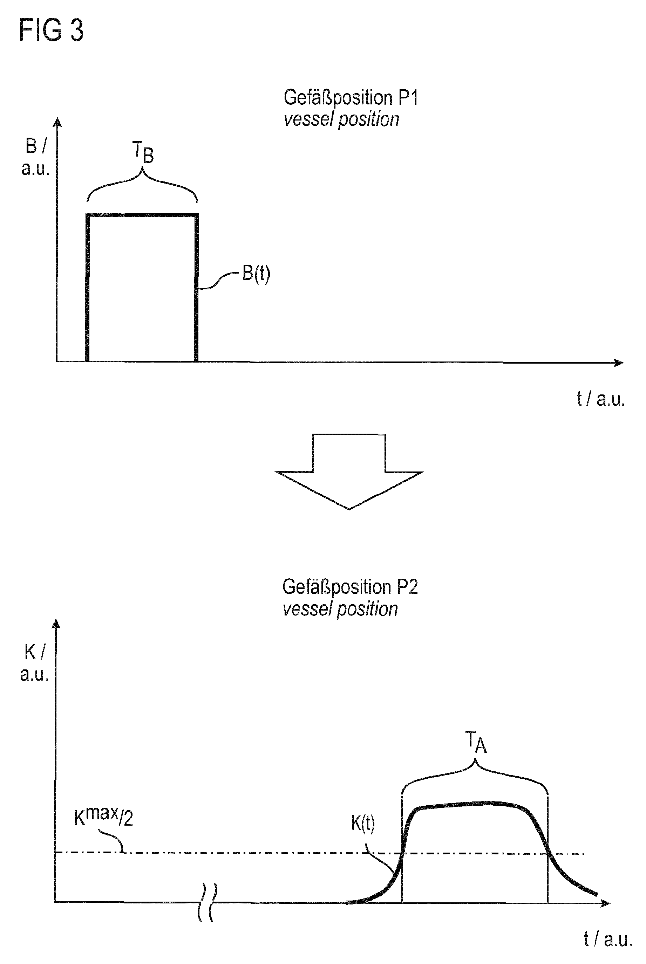

Eine Bolusapplikation mit Kontrastmittel, die an einer ersten Gefäßposition P1 appliziert wird hat meist einen Bolusverlauf B(t), wie er in der **Figur 3** im oberen Teil eingezeichnet ist. Dort ist der Volumenfluss des Bolus B mit einer Bolusapplikationsdauer T_{B} über die Zeit t aufgetragen, wobei als Kriterium zur Bestimmung der Bolusantwortdauer das Überschreiten des Kontrastmittelkonzentrationsverlaufes K(t) über ein Kontrastmittelkonzentrationsniveau, hier den halben Maximalwert K^{max}/2 zur Bestimmung der Halbwertsbreite, angesehen wird. Ein solcher Bolusverlauf B(t) kann - wie hier dargestellt einen rechteckigen Verlauf annehmen, kann jedoch auch durch entsprechende Einstellung an einem Kontrastmittelinjektor eine beliebige Funktion nachbilden. Ein solcher Volumenfluss B wird abhängig vom verwendeten Kontrastmittel (z.B. Gadovist® oder Magnevist®) und spezifischen Patienteneigenschaften gewählt, um am Ort der Untersuchung im darzustellenden Gefäß eine gewünschte Kontrastmittelkonzentration zu erreichen. Wie in der Figur 2 gezeigt passiert ein solcher Bolus nach der Applikation im venösen Gefäßbereich zumindest das Herz zweimal und die Lunge mit einer zwischendurch starken Verästelung der Gefäße. Beispielhaft ist unten in der Figur 3 der Kontrastmittelkonzentrationsverlauf K(t) einer Bolusantwort mit einer Bolusantwortdauer T_{A} an einer Gefäßposition P2 gezeigt.

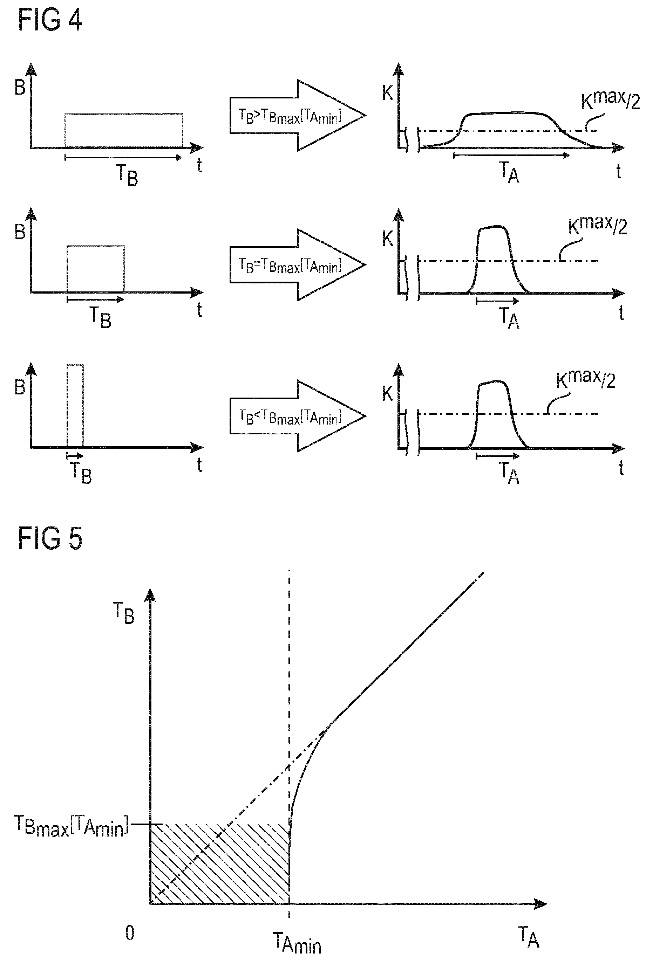

Drei unterschiedliche und typische Werte einer Bolusapplikationsdauer T_{B} im Vergleich zu den sich daraus ergebenden Werten für die jeweils resultierende Bolusantwortdauer T_{A} sind in der **Figur 4** dargestellt. Grundsätzlich existiert ein Proportionalitätsbereich für die Bolusapplikationsdauer T_{B} in dem eine größer beziehungsweise kleiner werdende Bolusapplikationsdauer T_{B} auch zu entsprechend größer beziehungsweise kleiner werdender Bolusantwortdauer T_{A} führt. Zu kürzer werdenden Zeiten der Bolusapplikationsdauer T_{B} erreicht die Bolusantwortdauer T_{A} einen Grenzwert einer minimal erreichbaren Bolusantwortdauer T_{Amin} der durch eine noch so kurze Bolusapplikationsdauer T_{B} nicht zu unterschreiten ist. Bedingt ist diese Situation insbesondere durch die Herz-Lungen-Passage und zusätzlich auftretende Turbulenzen, nicht-laminare Strömungen und Geschwindigkeitsdifferenzen über den Gefäßquerschnitt einschließlich durch Verdünnungseffekte im venösen System, die eine mehr oder minderstarke Dispersion des applizierten Kontrastmittelbolus erzeugen. Mutmaßlich sind hierbei Verdünnungseffekte sowohl in den Herzkammern, als auch im großen Lungenleervolumen wesentlich. Das Herz-Lungensystem verhält sich dabei wie ein Blutreservoir, in das ein kleines Kontrastmittelvolumen schnell eingebracht und dann verdünnt über einen längeren Zeitraum ausgestoßen wird.

In der **Figur 5** ist eine typische Beziehung zwischen der Dauer einer Bolusapplikation T_{B} an einer ersten Gefäßposition und der daraus resultierenden Dauer eine Bolusantwort T_{A} gegeneinander aufgetragen. Wie zu erkennen ist, verläuft die Kurve T_{A}(T_{B}) von rechts kommend zunächst etwa proportional im etwa 40°-Winkel zu kleineren Werten der Bolusapplikationsdauer T_{B} hin. Es folgt darauf ein gekrümmte Bereich, in dem der auftretende Verbreiterungseffekt beginnt zu dominieren, gefolgt von einem Grenzwert für die Bolusantwortdauer T_{Amin}, unter den eine Bolusantwortdauer - unter der Voraussetzung ansonsten gleicher Bedingungen - auch bei beliebig kurzer Bolusapplikation nicht verkürzt werden kann. Entsprechend ist auch ein Maximalwert einer Bolusapplikationsdauer T_{Bmax}[T_{Amin}] zu bestimmen, ab dem die minimale Bolusantwortdauer T_{Amin} erreicht wird.

Die hier beschriebenen Verhältnisse zwischen maximaler Bolusapplikationsdauer T_{Bmax}[T_{Amin}] zu Erreichung einer minimalen Bolusantwortdauer T_{Amin} gelten unter grundsätzlich gleichen Strömungsverhältnissen, die wiederum gleiche Blutflussparameter erzeugen. Gemäß der Erkenntnis der Erfinder lässt sich allerdings auch eine Korrelation oder eindeutige Beziehung zwischen solchen auf Blutflusseigenschaften basierenden Blutflussparametern und der oben beschriebenen maximalen Bolusapplikationsdauer T_{Bmax}[T_{Amin}] finden. Entsprechend kann unter der Voraussetzung eines vergleichbaren Patientenklientels, also Patienten mit weitgehend übereinstimmenden Patientenparametern, durch Bestimmung der Blutflussparameter die maximale Bolusapplikationsdauer T_{Bmax}[T_{Amin}] für gegebene Applikationspositionen des Bolus und Beobachtungspositionen der Bolusantwort vorhersagen, welche zu einer minimal möglichen Bolusantwortdauer T_{Amin} führt.

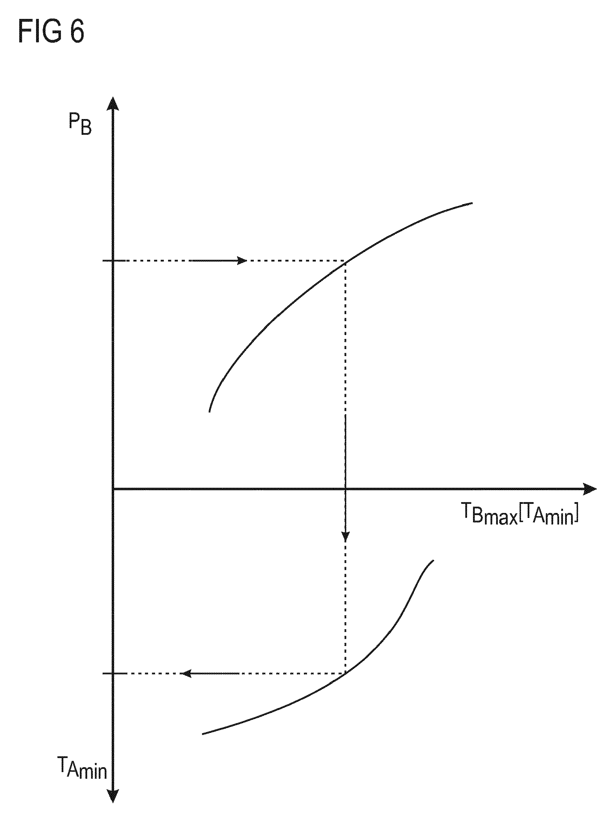

Die **Figur 6** zeigt beispielhaft die ermittelte Korrelation zwischen dem Blutflussparameter P_{B} entsprechend einer mittleren Flussgeschwindigkeit des Blutes an einer typischen Gefäßposition P3 eines Patientenkollektivs mit ähnlichen bis gleichen Patientenparametern Geschlecht, Gewicht, Größe, Alter, Herzfrequenz und BMI einerseits und der unter diesen Bedingungen ermittelten maximalen Bolusapplikationsdauer T_{Bmax}[T_{Amin}] für eine erste Gefäßposition P1 einer Bolusapplikation und Beobachtungspositionen der Bolusantwort an einer zweiten Gefäßposition P2 vorhersagen, welche zu einer minimal möglichen Bolusantwortdauer T_{Amin} führt. Entsprechend der graphischen Darstellung Lässt sich aus dem Diagramm der Figur 6 ausgehend von einem bei einem zu untersuchenden Patienten, dessen Patientenparameter mit dem hier verwendeten Patientenkollektiv übereinstimmt zunächst ein Wert auf der oberen Kurve ablesen, der zu einer maximalen Bolusantwortdauer T_{Bmax}[T_{Amin}] auf der Abszisse führt. Von dort weiter senkrecht nach unten gehend ergibt sich ein Wert auf der unteren Kurve, von dem aus horizontal nach links zur Ordinate gehend der Wert für die unter diesen Bedingungen erreichbare minimale Bolusantwortdauer T_{Amin} abgelesen werden kann.

Selbstverständlich können die hier graphisch abgebildeten Korrelationen auch über eine entsprechende Look up-Tabelle (LUT) oder entsprechende mathematischen Funktionen beschrieben werden.

Insgesamt wird mit der Erfindung also ein Verfahren zur Vorbestimmung einer maximalen - im Sinne einer maximal möglichen - Bolusapplikationsdauer zur Erreichung einer minimal möglichen Bolusantwortdauer einer Kontrastmittelbolusantwort an einer zweiten Gefäßposition in einer Untersuchungsumgebung eines Patienten an einer ersten Gefäßposition für ein kontrastmittelgestütztes bildgebendes Aufnahmeverfahren vorgeschlagen, wobei hierfür eine Korrelation zwischen der maximal möglichen Bolusapplikationsdauer für eine Patientengruppe mit bekannten Patientenparametern und mindestens einem Blutflussparameter ermittelt wird.

Außerdem wird auch ein Verfahren zur Bestimmung einer optimalen Flussrate für eine kürzest mögliche Bolusapplikation vorgeschlagen, wobei die maximal erreichbare Bolusapplikationsdauer bei einer gleichzeitig kürzest möglichen Bolusantwortdauer ermittelt, und hieraus die dafür notwendige Flussrate zur Applikation einer vorgegebenen zu applizierenden Kontrastmittelmenge bestimmt wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen. Insbesondere beschränkt sich die Erfindung nicht auf die hier angegebenen Merkmalskombinationen, sondern es können auch für den Fachmann offensichtlich ausführbare andere Kombinationen und Teilkombination aus den offenbarten Merkmalen gebildet werden. Des Weiteren beschränkt sich die Erfindung nicht auf die in den Ansprüchen verwendeten Anspruchskategorien, sondern umfasst auch die offenbarten Merkmale in Verbindung mit allen dem Fachmann geläufigen sonstigen Anspruchskategorien.

### Bezugszeichenliste

- 1: Magnetresonanztomographie-System (MRT-System)
- 2: Magnetspulen
- 3: Empfangsspule
- 4: Magnetspulen
- 5: Kontrastmittelinjektor
- 6: Gehäuse
- 7: Patient
- 8: Steuer- und Recheneinheit / Computer mit Speicher und Display
- 9: Computer-Programme
- B: Bolus
- B(t): zeitlicher Bolusverlauf
- K: Kontrastmittelkonzentration
- K(t): zeitlicher Kontrastmittelkonzentrationsverlauf / Kontrastmittelbolusantwort
- K^{max}/2: halber Maximalwert der Kontrastmittelkonzentration
- P_{B}: Blutflussparameter
- P1: erste Gefäßposition
- P2: zweite Gefäßposition
- P3: dritte Gefäßposition
- P4: vierte Gefäßposition
- ROI: Untersuchungsgebiet
- t: Zeit
- T_{A}: Bolusantwortdauer
- T_{B}: Bolusapplikationsdauer
- T_{Bmax}[T_{Amin}]: maximale Bolusapplikationsdauer zur Erreichung einer minimalen Bolusantwortdauer
- T_{Amin}: minimale Bolusantwortdauer

## Patentansprüche

1. Verfahren zur Vorbestimmung einer maximalen Bolusapplikationsdauer (T_{Bmax}[T_{Amin}]) zur Erreichung einer minimalen Bolusantwortdauer (T_{Amin}) einer Kontrastmittelbolusantwort (K(t)) an einer zweiten Gefäßposition (P2) in einer Untersuchungsumgebung (ROI) eines Patienten (P) nach einer automatisierten Bolusapplikation (B(t)) mit einem Kontrastmittelapplikator (5) an einer ersten Gefäßposition (P1) für ein kontrastmittelgestütztes bildgebendes Aufnahmeverfahren, wobei die folgenden Verfahrensschritte ausgeführt werden:
1.1. Ermittlung einer Korrelation zwischen der maximalen Bolusapplikationsdauer (T_{Bmax}[T_{Amin}]) für eine Patientengruppe mit mindestens einem bekannten Patientenparameter (P_{P}) einerseits und mindestens einem Blutflussparameter (P_{B}) andererseits, wobei der Blutflussparameter (P_{G}) von mindestens einer Blutflusseigenschaft (E_{B}) an einer dritten Gefäßposition (P3) abhängig ist,
1.2. Ausgabe einer Tabelle (LUT) oder Funktion der Korrelation und/oder Speicherung einer Tabelle oder Funktion der Korrelation in einem elektronischen Speicher zur weiteren Verwendung durch einen Computer.

2. Verfahren gemäß dem voranstehenden Anspruch **1,dadurch g e - kennzeichnet,** dass die zweite Gefäßposition (P2) und/oder die Untersuchungsumgebung (ROI) sich strömungsbezogen hinter der Herz-Lungen-Passage befindet.

3. Verfahren gemäß einem der voranstehenden Ansprüche 1 bis 2, **dadurch gekennzeichnet, dass** die mindestens eine Blutflusseigenschaft (E_{B}) unter Abwesenheit von Kontrastmittel durch eine Phasenkontrast-Magnetresonanz-Messung ermittelt wird.

4. Verfahren gemäß einem der voranstehenden Patentansprüche 1 bis 3, **dadurch gekennzeichnet, dass** mindestens ein Patientenparameter (P_{P}) der nachfolgenden Liste verwendet wird:
- Geschlecht,
- Gewicht,
- Größe,
- Alter,
- Herzfrequenz,
- BMI,
- Typisierung der Statur,
- Abstand zwischen vorgegebenen Gefäßpositionen.

5. Verfahren gemäß einem der voranstehenden Patentansprüche 1 bis 4, **dadurch gekennzeichnet, dass** als mindestens eine Blutflusseigenschaft (E_{B}) mindestens eine der folgenden Eigenschaften verwendet wird:
- maximale, minimale oder mittlere Blutflussgeschwindigkeit (v_{G}) an mindestens einer vorgegebenen Position im Gefäßquerschnitt an der Gefäßposition;
- maximale, minimale oder mittlere Blutflussgeschwindigkeit (v_{G}) an mindestens einer vorgegebenen Position im Gefäßquerschnitt an der Gefäßposition zu einer vorgegebenen Herz- oder Pulsphase;
- maximale, minimale oder mittlere Blutflussgeschwindigkeit (v_{G}) an mindestens einer vorgegebenen Position im Gefäßquerschnitt an der Gefäßposition zu einer vorgegebenen Herz- oder Pulsphase über einen vorgegebenen Messzeitraum;
- maximales, minimales oder mittleres Blutflussvolumen an mindestens einer vorgegebenen Position im Gefäßquerschnitt an der Gefäßposition;
- maximale, minimale oder mittlere Blutflussvolumen an mindestens einer vorgegebenen Position im Gefäßquerschnitt an der Gefäßposition zu einer vorgegebenen Herz- oder Pulsphase;
- maximale, minimale oder mittlere Blutflussvolumen an mindestens einer vorgegebenen Position im Gefäßquerschnitt an der Gefäßposition zu einer vorgegebenen Herz- oder Pulsphase über einen vorgegebenen Messzeitraum;
- eine geometrische Eigenschaft eines Geschwindigkeitsprofils über den Gefäßquerschnitt an der Gefäßposition;
- Nettovorwärtsvolumen über einen vorgegebenen Zeitraum oder pro Herzschlag.

6. Verfahren gemäß einem der voranstehenden Patentansprüche 1 bis 5, **dadurch gekennzeichnet, dass** als Blutflussparameter (P_{B}) die Blutflusseigenschaft (E_{B}) selbst verwendet wird

7. Verfahren gemäß einem der voranstehenden Patentansprüche 1 bis 5, **dadurch gekennzeichnet, dass** als Blutflussparameter (P_{B}) eine absolute oder prozentuale Differenz zwischen der Blutflusseigenschaft (E_{B}) an der dritten Gefäßposition (P3) zur gleichen Blutflusseigenschaft (E_{B}) an einer vierten Gefäßposition (P4) verwendet wird.

8. Verfahren gemäß einem der voranstehenden Patentansprüche 1 bis 7, **dadurch gekennzeichnet, dass** die erste Gefäßposition (P1) mindestens eine der nachfolgenden Ortsangaben oder Bedingungen erfüllt:
- die erste Gefäßposition (P1) liegt in einem venösen Gefäß,
- die erste Gefäßposition (P1) liegt in einer Armvene,
- die erste Gefäßposition (P1) liegt am Handrücken,
- die erste Gefäßposition (P1) liegt an einem zentralvenösen Katheter,
- die erste Gefäßposition (P1) liegt zwischen Handrücken und V. axillari,
- die erste Gefäßposition (P1) liegt zwischen Fuß und V. saphena magna
- die erste Gefäßposition (P1) liegt in einem zentralvenösen Gefäß.

9. Verfahren gemäß einem der voranstehenden Patentansprüche 1 bis 8, **dadurch gekennzeichnet, dass** die zweite Gefäßposition (P2) mindestens eine der nachfolgenden Ortsangaben oder Bedingungen erfüllt:
- die zweite Gefäßposition (P2) liegt in einem arteriellen Gefäß,
- die zweite Gefäßposition (P2) liegt in einer Beinarterie,
- die zweite Gefäßposition (P2) liegt flussabwärts zur dritten Gefäßposition (P3),
- die zweite Gefäßposition (P2) liegt flussabwärts der Bifurcation,
- die zweite Gefäßposition (P2) liegt in einer peripheren Arterie, vorzugsweise im Kniebereich,
- die zweite Gefäßposition (P2) liegt im Armbereich,
- die zweite Gefäßposition (P2) liegt im Kopfbereich
- die zweite Gefäßposition (P2) liegt im Halsbereich.

10. Verfahren gemäß einem der voranstehenden Patentansprüche 1 bis 9, **dadurch gekennzeichnet, dass** die dritte Gefäßposition (P3) mindestens eine der nachfolgenden Ortsangaben oder Bedingungen erfüllt:
- die dritte Gefäßposition (P3) ist die zweite Gefäßposition (P2),
- die dritte Gefäßposition (P3) liegt in der Aorta thorakalis,
- die dritte Gefäßposition (P3) liegt in der Aorta abdominalis,
- die dritte Gefäßposition (P3) liegt in der Aorta ascendens,
- die dritte Gefäßposition (P3) liegt zwischen Aorta thorakalis und (P2)
- die dritte Gefäßposition (P3) liegt vor der Bifurcation,
- die dritte Gefäßposition (P3) liegt zwischen Bifurcation und (P2).

11. Verfahren gemäß einem der voranstehenden Patentansprüche 7 bis 10, **dadurch gekennzeichnet, dass** die vierte Gefäßposition (P4) mindestens eine der nachfolgenden Ortsangaben oder Bedingungen erfüllt:
- die vierte Gefäßposition (P4) liegt flussaufwärts der dritten Gefäßposition (P3),
- die vierte Gefäßposition (P4) liegt flussabwärts zwischen der dritten Gefäßposition (P3) und der zweiten Gefäßposition (P2),
- die vierte Gefäßposition (P4) liegt flussabwärts der zweiten Gefäßposition (P2).

12. Verfahren zur Bestimmung einer optimalen Flussrate (F) für eine kürzest mögliche automatisierte Bolusapplikation eines Kontrastmittels (KM) mit einem Kontrastmittelapplikator (5) für eine kontrastmittelgestützte Aufnahme eines Patienten (P), **dadurch gekennzeichnet, dass** zumindest die folgenden Verfahrensschritte ausgeführt werden:
12.1. Ermittlung einer maximalen Bolusapplikationsdauer (T_{Bmax}[Tₘᵢₙ]) zur Erreichung einer minimalen Bolusantwortdauer (T_{Amin}) einer Kontrastmittelbolusantwort (A_{P2}) an einer zweiten Gefäßposition (P2) in einer Untersuchungsumgebung (ROI) eines Patienten (P) nach einer automatisierten Bolusapplikation (B(t)) mit einem Kontrastmittelapplikator (5) an einer ersten Gefäßposition (P1),
12.2. Berechnung einer an einem Kontrastmittelinjektor (5) einzustellenden Flussrate (F) für einen Kontrastmittelbolus bei der vorbestimmten maximalen Bolusapplikationsdauer (T_{Bmax}[T_{Amin}]) zur Applikation einer vorgegebenen zu applizierenden Kontrastmittelmenge (M_{KM}),
12.3. Speicherung der berechneten Flussrate (F) und der Bolusapplikationsdauer (T_{B}= T_{Bmax}[T_{Amin}]) in einem Speicher des Kontrastmittelinjektors (5) zur Verwendung für eine auszuführende Kontrastmittelbolusinjektion.

13. Verfahren gemäß dem voranstehenden Patentanspruch 12, **dadurch gekennzeichnet, dass** zur Ermittlung der Bolusapplikationsdauer (T_{B=} T_{Bmax}[T_{Amin}]) zumindest die folgenden Verfahrensschritte ausgeführt werden:
13.1. Durchführung einer kontrastmittelfreien Messung mindestens einer Blutflusseigenschaft (E_{B}) am aktuell zu untersuchenden Patienten (P),
13.2. Bestimmung mindestens eines Blutflussparameters (P_{B}) auf der Basis der gemessenen mindestens einen Blutflusseigenschaft (E_{B}),
13.3. Ermittlung der maximalen Bolusapplikationsdauer (T_{Bmax}[T_{Amin}]) zur Erreichung einer minimalen Bolusantwortdauer (T_{Amin}) unter Anwendung einer vorbestimmten Korrelation zwischen dem mindestens einen Blutflussparameter (P_{B}) und der maximalen Bolusapplikationsdauer (T_{Bmax}[T_{Amin}]) unter Berücksichtigung mindestens eines Patientenparameters (P_{P}).

14. Verfahren gemäß dem voranstehenden Patentanspruch 13, **dadurch gekennzeichnet, dass** die vorbestimmte Korrelation gemäß einem der Patentansprüche 1 bis 11 ermittelt wurde.

15. Verfahren gemäß dem voranstehenden Patentanspruch 14, **dadurch gekennzeichnet, dass** zur Bestimmung der maximalen Bolusapplikationsdauer (T_{Bmax}[T_{Amin}]) die gleichen Blutflussparameter (P_{B}) und Patientenparameter (P_{P}) am aktuellen Patienten (P) an den gleichen Gefäßpositionen (P1 bis P4) ermittelt werden, die auch zur Ermittlung der Korrelation gemäß einem der Patentansprüche 1 bis 11 verwendet wurden.

16. Verfahren gemäß einem der voranstehenden Patentansprüche 12 bis 15, **dadurch gekennzeichnet, dass** zusätzlich die folgenden Verfahrensschritte ausgeführt werden:
16.1. Applikation des Kontrastmittels mit einem Kontrastmittelapplikator (5) unter Anwendung der ermittelten Flussrate (F) über die vorgegebene Bolusapplikationsdauer (T_{B}= T_{Bmax}[T_{Amin}]),
16.2. Erzeugung zumindest einer Abbildung zumindest der Untersuchungsumgebung (ROI) des Patienten (P) durch ein kontrastmittelgestütztes bildgebendes Verfahren.

17. Verfahren gemäß dem voranstehenden Patentanspruch 16, **dadurch gekennzeichnet, dass** die Erzeugung zumindest der mindestens einen Abbildung durch eine Magnetresonanz-Untersuchung oder mit einem Magnetresonanztomographie-System erfolgt.

18. Verfahren gemäß einem der voranstehenden Patentansprüche 12 bis 17, **dadurch gekennzeichnet, dass** die kontrastmittelgestützte Erzeugung einer Aufnahme des Untersuchungsgebietes (ROI), vorzugsweise im Kopf-/Gehirnbereich, nur während der ersten Anflutung des Kontrastmittels (KM) im Untersuchungsgebiet erfolgt.

19. Verfahren zur Vermeidung einer fehlerhaften Einstellung der Flussrate (F) und/oder Bolusapplikationsdauer (T_{B}) eines Kontrastmittelapplikators (5), der einen Prozessor und einen Speicher aufweist oder damit verbunden ist, um mindestens ein im Speicher vorliegendes Computerprogramm im Betrieb auszuführen, **dadurch gekennzeichnet, dass** das mindestens eine Computerprogramm eine Ablaufroutine aufweist, durch welche sichergestellt wird, dass eine eingegebene Bolusapplikationsdauer (T_{B}) gleich oder größer als eine zuvor ermittelte maximale Bolusapplikationsdauer (T_{Bmax}[T_{Amin}]) zur Erreichung einer minimalen Bolusantwortdauer (T_{Amin}) einer Kontrastmittelbolusantwort (A_{P2}) an einer zweiten Gefäßposition (P2) in einer Untersuchungsumgebung (ROI) eines Patienten (P) nach einer automatisierten Bolusapplikation (B(t)) mit einem Kontrastmittelapplikator (5) an einer ersten Gefäßposition (P1) ist.

20. Verfahren gemäß dem voranstehenden Patentanspruch 19, **dadurch gekennzeichnet, dass** die maximale Bolusapplikationsdauer (T_{Bmax}[T_{Amin}]) zur Erreichung einer minimalen Bolusantwortdauer (T_{Amin}) mit den gleichen Blutflussparametern (P_{B}) und Patientenparametern (P_{P}) am aktuellen Patienten (P) an den gleichen Gefäßpositionen ermittelt wird, die auch zur Ermittlung der Korrelation gemäß einem der Patentansprüche 1 bis 11 verwendet werden.

21. Verfahren gemäß einem der voranstehenden Patentansprüche 19 bis 20, **dadurch gekennzeichnet, dass** die maximale Bolusapplikationsdauer (T_{Bmax}[T_{Amin}]) zur Erreichung einer minimalen Bolusantwortdauer (T_{Amin}) des aktuell untersuchten Patienten (P) mit Hilfe einer kontrastmittelfreien Phasenkontrast-Magnetresonanz-Bestimmung mindestens einer Blutflusseigenschaft (E_{B}), vorzugsweise mit den Merkmalen eines der Ansprüche 12 bis 15, ermittelt wird.

22. Magnetresonanzsystem (1) zur Erzeugung einer kontrastmittelunterstützten MR-Aufnahme mit einem, von einem prozessorgesteuerten Kontrastmittelinjektor (5), wobei Computer und ein Speicher für Computerprogramme vorliegen, **dadurch gekennzeichnet, dass** Computerprogramme gespeichert sind, welche im Betrieb das Verfahren gemäß einem der voranstehenden Verfahrensansprüche ausführen.
